# EUROPÄISCHE PATENTSCHRIFT

(11) **EP 0 406 743 B1**
(45) Veröffentlichungstag und Bekanntmachung des Hinweises auf die Patenterteilung: **07.12.1994**
(21) Anmeldenummer: 90112542.7
(22) Anmeldetag: 30.06.1990
(51) Int. Cl.: A61K 31/535

(54) **N-Benzyl-N-((1S,5S)-6,6-dimethylbicyclo [3.1.1]hept-2-yl-äthoxy-äthyl)-morpholinium-Salze enthaltende gastroprotektiv wirksame pharmazeutische Zubereitungen**
Gastroprotective pharmaceutical preparations containing N-benzyl-N-((1S,5S)-6,6-dimethylbicyclo[3.1.1]hept-2-yl-ethoxy-ethyl)-morpholinium salts
Compositions parmaceutiques gastroprotectrices contenant des sels de N-benzyl-N-((1S,5S)-6,6-diméthylbicyclo[3.1.1]hept-2-yl-éthoxy-éthyl)-morpholinium

(30) Priorität: 07.07.1989 DE 3922387
(43) Veröffentlichungstag der Anmeldung: 09.01.1991
(73) Patentinhaber: Kali-Chemie Pharma GmbH, D-30173 Hannover (DE)
(72) Erfinder: Christen, Marie-Odile, F-75016 Paris (FR); Noel, Brigitte, F-37320 Truyes (FR); Philippi, Ilse, D-3006 Burgwedel 1 (DE)
(74) Vertreter: Lauer, Dieter, Dr.

(56) Entgegenhaltungen:
- FR-A- 2 097 032
- MED.INT., Band 13, Nr. 12, Dezember 1978; A.ROBERTI et al., Seiten 654-656 #
- REVUE MEDICALE DE LIEGE, Band 36, Nr. 13-14, Juli 1981; C.BATAILLE et al.,Seiten 49-52
- Naunyn-Schmiedeberg's Arch. Pharmacol., Band 323, 1983, Seiten 72-77, Springer-Verlag; G. Droogmans et al.: "Effect of pinaverium bromide on electrical and mechanical activity of smooth muscle cells"
- Bordeaux Medical, Band 10, Nr. 21, 1977, Seiten 1457-1459; J.-J. Dubarry et al.: "Effect à court terme du bromure de pinavérium dans les oesophagites, gastro-duodénites et colopathies fonctionnelles"

## Beschreibung

Die vorliegende Erfindung betrifft die Verwendung von quartären N-Benzyl-N-{2-[2-((1S,5S)-6,6-dimethylbicyclo[3.1.1]hept-2-yl)-äthoxy]-äthyl}-morpholinium-Salzen zur Prophylaxe und Behandlung von Blutzirkulationsstörungen im peripheren, insbesondere im mikrovaskulären Bereich der Magen-/Darmwände und von Schädigungen des gastrointestinalen Traktes in größeren Säugetieren, insbesondere Menschen, welche durch gastrotoxische Dosen von Arzneimitteln oder Chemikalien verursacht werden. Insbesondere betrifft die Erfindung die Prophylaxe und Behandlung von Schädigungen des Magen/Darmtraktes, welche durch die oftmals chronische Einnahme von nichtsteroidalen antiinflammatorischen Arzneimitteln zum Beispiel in der Rheumatherapie verursacht werden können.

Zu den nichtsteroidalen Antiphlogistika und Antirheumatika (= non steroid antiinflammatory drugs im folgenden abgekürzt als NSAIDs) gehören unter anderem substituierte Benzoesäurederivate, beispielsweise Salicylsäurederivate wie z.B. Acetylsalicylsäure (= Aspirin^{R}) oder Salicylamid; Anthranilsäurederivate wie z.B. Flufenaminsäure; Arylessigsäurederivate, beispielsweise substituierte Phenylessigsäurederivate wie z.B. Diclofenac oder Ibufenac, substi tuierte Indolessigsäurederivate wie z.B. Indomethacin oder substituierte 2-Phenylpropionsäurederivate wie z.B. Ibuprofen; Chinolincarbonsäurederivate; Pyrazolindionderivate, beispielsweise Diphenylpyrazolindionderivate wie z.B. Phenylbutazon oder Oxyfenbutazon; Phenylpyrazolonderivate wie z.B. Phenazon, Aminophenazon oder Propiphenazon; Oxicamderivate wie z.B. Piroxicam oder Tenoxicam.

Es ist bekannt, daß NSAIDs Cyclooxygenase-inhibitorische Wirkungen haben und somit hemmend auf die endogene Prostaglandinbildung wirken. Prostaglandine finden sich in verhältnismäßig hohen Konzentrationen in den Wänden des gastrointestinalen Traktes. Sie spielen eine wichtige Rolle in der gastrointestinalen Physiologie und beeinflussen eine Vielzahl von Funktionen im Magen/Darmtrakt. So zeigen die Prostaglandine zum Beispiel neben säuresekretionshemmenden Wirkungen auch die Durchblutung der Magenschleimhaut fördernde und mucosaprotektive Eigenschaften.

NSAIDs können bekanntermaßen, insbesondere bei Langzeitanwendung, an den Schleimhäuten und Wandungen des Magen/Darmtraktes zu schwerwiegenden Schädigungen, z.B. Hämorrhagien bis hinzu schwersten Magenblutungen und Läsionen führen.

Die als Ulcustherapeutika sonst erfolgreich eingesetzten H₂-rezeptorantagonistisch wirksamen Substanzen vermögen die durch NSAIDs induzierten Schädigungen im Magen/Darmtrakt nicht wirkungsvoll zu verhindern.

Aufgabe der vorliegenden Erfindung ist es, Arzneimittel zur Prophylaxe und zur Behandlung von Störungen der peripheren, insbesondere der mikrovaskulären Durchblutung der Magen-/Darmwände und von durch gastrotoxische Dosen von Arzneimitteln, insbesondere NSAIDs, und Chemikalien induzierten Schädigungen der Magen-/Darmwände zu entwickeln.

Quartäre N-Benzyl-N-{2-[2-((1S, 5S)-6,6-dimethylbicyclo[3.1.1]hept-2-yl)-äthoxy]-äthyl}-morpholinium-Salze mit spasmolytischen Eigenschaften sind aus dem französischen Patent Nr. 2097032 bzw. dem deutschen Patent Nr. 2137988 bekannt. Das unter den Umfang der vorgenannten Patente fallende Pinaveriumbromid (= N-(2-Brom-4,5-dimethoxybenzyl)-N-{2-[2((1S,5S)-6,6-dimethylbicyclo[3.1.1]hept-2-yl)-äthoxy]-äthyl}-morpholiniumbromid) ist beispielsweise unter dem Handelsnamen Dicetel^{R} als Spasmolytikum im Handel.

Erfindungsgemäß werden quartäre N-Benzyl-N-{2-[2-((1S,5S)-6,6-dimethylbicyclo[ 3.1.1]hept-2-yl)-äthoxy]-äthyl}-morpholinium-Salze der allgemeinen Formel I
(s. Formel I)
worin
- R¹: Wasserstoff, Halogen, niederes Alkyl oder niederes Alkoxy bedeutet,
- R²: Wasserstoff, Halogen, niederes Alkyl oder niederes Alkoxy bedeutet,
- R³: Wasserstoff, Halogen, niederes Alkyl oder niederes Alkoxy bedeutet und
- X⁻: das Anion einer pharmakologisch akzeptablen Säure darstellt,
verwendet zur Herstellung von pharmazeutischen Zübereitungen zur Prophylaxe und Behandlung von Blutzirkulationsstörungen im peripheren, insbesondere mikrovaskulären Bereich der Magen-/Darmwände und von durch gastrotoxische Dosen von Arzneimitteln, insbesondere nichtsteroidalen entzündungshemmenden Arzneimitteln, oder Chemikalien induzierten Schädigungen der Magen-/Darmwände in größeren Säugetieren und Menschen.

In den Salzen der Formel I stellt X⁻ das Anion einer physiologisch verträglichen anorganischen oder organischen Säure dar. Als Anionen anorganischer Säuren eignen sich z.B. Halogenide, insbesondere Bromid und Chlorid, aber auch Sulfate oder Phosphate. Als organische Säuren eignen sich z.B. niedere aliphatische oder aromatische Sulfonsäuren, insbesondere Niederalkylsulfonsäuren, wie Methansulfonsäure oder gegebenenfalls im Benzolring durch niederes Alkyl oder Halogen substituierte Benzolsulfonsäuren wie Toluolsulfonsäuren.

Sofern in den Verbindungen der Formel I die Substituenten R¹ bis R³ niedere Alkyl- oder Alkoxygruppen darstellen, können diese geradkettig oder verzweigt sein und 1 bis 4, vorzugsweise 1 bis 2, Kohlenstoffatome enthalten und insbesondere Methyl oder Methoxy bedeuten. Sofern die Substituenten R¹ bis R³ Halogen darstellen, kommen Fluor, Chlor oder Brom, insbesondere Brom, in Frage.

Als geeignet erweisen sich insbesondere Pinaverium-Salze, beispielsweise das Pinaverium-Bromid (Handelsprodukt Dicetel^{R}).

Die Verbindungen der Formel I sind 2-(6,6-Dimethylbicyclo[3.1.1]hept-2-yl)-äthanolderivate, welche in den Positionen 1, 2 und 5 des Bicycloheptanring-Gerüstes (s. Formel I) chirale Zentren enthalten, welche jeweils R- oder S-konfiguriert sein können. Die in den Verbindungen der Formel I enthaltene 2-(6,6-Dimethylbicyclo[3.1.1]hept-2-yl)-äthoxy-Gruppierung stammt von einem Terpenalkohol, welcher sich von dem natürlichen (-)-β-Pinen (= (1S,5S-(-)-6,6-Dimethyl-2-methylenbicyclo[3.1.1]heptan) ableitet. In diesem Terpenalkohol besitzen die chiralen Zentren in 1- und 5-Position S-Konfiguration. Dementsprechend sind auch in den 2-[2-((1S,5S)-6,6-dimethylbicyclo[3.1.1]hept-2-yl)-äthoxy]-äthanolderivaten der Formel I die chiralen Zentren in 1- und 5-Position des Ringgerüstes S-konfiguriert, während das chirale Zentrum in 2-Position S- oder R-Konfiguration besitzen kann. So können die Substanzen der Formel I in zwei diastereoisomeren Formen auftreten. Erfindungsgemäß können die einzelnen stereoisomeren Formen der Verbindungen der Formel I oder deren Gemische verwendet werden.

Die Verbindungen der Formel I fallen unter den Umfang des französischen Patentes Nr. 2097032. Sie können nach oder analog zu den in diesem französischen Patent beschriebenen Methoden hergestellt werden. Beispielsweise werden die Verbindungen der Formel I auf an sich bekannte Weise erhalten durch Quarternisierung von Verbindungen der Formel II
(s. Formel II)
mit Benzylhalogeniden der Formel III
(s.Formel III)
worin R¹, R² und R³ obige Bedeutung besitzen, und Y Halogen bedeutet.

Verbindungen der Formel II können auf an sich bekannte Weise; z.B. nach oder analog zu den in dem französischen Patent Nr. 2097031 beschriebenen Methoden, ausgehend von dem ungesättigten Terpenalkohol der Formel IV
(s. Formel IV)
erhalten werden, indem man diesen zunächst zu dem entsprechenden gesättigten Alkohol der Formel V
(s. Formel V)
hydriert und den Alkohol der Formel V anschließend mit einem Morpholinoäthylhalogenid der Formel VI
(s. Formel VI)
worin Y obige Bedeutung besitzt, umsetzt oder indem man den Alkohol der Formel IV zunächst mit der Verbindung der Formel VI umsetzt und das Reaktionsprodukt anschließend hydriert.

Bei dem aus natürlichem (-)-β-Pinen hergestellten Alkohol der Formel V und seinen Derivaten sind die chiralen Zentren in den Positionen 1 und 5 des Bicycloheptangerüstes S-konfiguriert, während das chirale Zentrum in 2-Position S- oder R-Konfiguration besitzen kann. Bei der Hydrierung des ungesättigten Alkohols der Formel IV oder seines Umsetzungsproduktes mit einer Verbindung VI entsteht ein Stereoisomerengemisch aus am chiralen Zentrum in 2-Stellung R- und S-konfigurierten Verbindungen, dessen Zusammensetzung je nach Art der Hydrierung variieren kann. Aus den Gemischen können die einzelnen stereoisomeren Formen gewünschtenfalls durch übliche Trennverfahren, z.B. fraktionierte Kristallisation geeigneter Salze oder chromatographische Verfahren angereichert und isoliert werden. Bei den weiteren Umsetzungen bleibt die Konfiguration am Bicycloheptangerüst erhalten. So werden je nach eingesetztem Ausgangsprodukt als Endprodukte der Formel I Stereoisomerengemische oder stereoisomerenreine Substanzen erhalten. Stereoisomerengemische können gewünschtenfalls auf an sich bekannte Weise aufgetrennt werden.

Es wurde überraschenderweise gefunden, daß die quartären N-Benzyl-N-{2-[2-((1S,5S)-6,6-dimethylbicyclo[3.1.1]hept-2-yl)-äthoxy]-äthyl}-morpholinium-Salze der Formel I außer ihren vorbekannten spasmolytischen Eigenschaften auch die Fähigkeit besitzen, Durchblutungsstörungen im Magen/Darmtrakt, insbesondere Blutstauungen im peripheren und mikrovaskulären Bereich der Magen-/Darmwände, und dem Auftreten von Stasen und Hämorrhagien entgegenzuwirken und im Magen/Darmtrakt eine Schutzwirkung auszuüben gegenüber schädigenden Einflüssen von gastrotoxischen Dosen von Arzneimitteln und anderen chemischen Substanzen, beispielsweise Alkohol, welche die Magen-/ Darmwände schädigen können, insbesondere solchen Arzneimitteln und Substanzen, deren pharmakologische Wirkungsprofile auch eine Behinderung der Prostaglandinsynthese umfassen. So erweisen sich die Salze der Formel I als wirksam zur Prophylaxe und Behandlung von durch NSAIDs, beispielsweise den vorstehend angeführten Antiphlogistika und Antirheumatika, verursachten Schädigungen der Magen-/ Darmwände.

Die Schutzwirkung der Verbindungen der Formel I gegenüber Hämorrhagien und NSAIDs-induzierten hämorrhagischen Läsionen im Magen/Darmtrakt lassen sich in pharmakologischen Standardtests an Tieren und in klinischen Studien nachweisen.

### Beschreibung der pharmakologischen Versuche.

### 1. Bestimmung der Hemmwirkung der Verbindungen gegen durch Aspirin^{R} induzierte Läsionen.

Es werden Gruppen von mindestens 6 männlichen Ratten mit einem Körpergewicht von 180-200 g pro Testdosis verwendet. Die Tiere werden 24 Stunden nüchtern gehalten, wobei ihnen Wasser unbegrenzt zur Verfügung steht. Die Prüfsubstanzen werden per os suspendiert in 0,5 ml Suspensionsmedium (1 %-ige Methylcelluloselösung) pro 100 g Tiergewicht appliziert. Eine Kontrollgruppe von Tieren erhält nur das entsprechende Volumen an Suspensionsmedium. Eine Stunde nach Applikation der Testsubstanzen werden den Tieren per os 200 mg/kg Acetylsalicylsäure ebenfalls suspendiert in 0,5 ml Suspensionsmedium pro 100 g Tiergewicht verabreicht. Die Tiere werden 5 Stunden nach der Aspirinapplikation getötet. Die Mägen werden entnommen und geöffnet und Anzahl und Größe der gebildeten Läsionen in der Schleimhaut werden beurteilt. Die Auswertung erfolgt modifiziert nach 0. Münchoff (Arzneim. Forsch. (Drug Res.) 4, 341-344 (1954)). Es werden Mittelwert und Standardabweichungen berechnet und daraus die Hemmwirkung der Prüfsubstanzen in % gegenüber der Kontrollgruppe bestimmt.

Die Ergebnisse werden in der nachfolgenden Tabelle I wiedergegeben.

**Tabelle I**

| Substanz | Dosis in µmol/kg p.o. | Hemmwirkung auf Aspirin^{R}-induzierte Läsionen im Rattenmagen % Hemmwirkung |
|---|---|---|
| Pinaveriumbromid¹) | 100 | 53 |
| | 215 | 88 |
| trans-Pinaveriumbromid²) | 100 | 61 |
| | 215 | 67 |
| cis-Pinaveriumbromid³) | 100 | 27 |
| | 215 | 70 |

| | | |
|---|---|---|
| ¹) Stereoisomeren-Gemisch | | |
| ²) Substituent in 2-Stellung transständig zur Dimethylmethylengruppe | | |
| ³) Substituent in 2-Stellung cisständig zur Dimethylmethylengruppe | | |

### 2. Bestimmung der Schutzwirkung der Substanzen gegenüber durch chemische Schadstoffe induzierte vaskuläre Schädigungen insbesondere hämorrhagische Läsionen im gastrointestinalen Trakt der Ratte.

Für die Versuche werden pro Testdosis und als Kontrolltiergruppe Gruppen von jeweils 3 bis 4 Sprague-Dawley-Ratten mit einem Körpergewicht von 160-200 g verwendet. Die Versuche werden zweimal wiederholt und die Ergebnisse gemittelt. Den nüchternen Ratten werden eine Suspension der Testdosis in Kochsalzlösung bzw. zur Kontrolle nur das Suspensionsmittel mittels einer Schlundsonde intragastral appliziert. Nach einer Zeitspanne von 5 oder 30 Minuten bzw. 1, 2 oder 6 Stunden werden den Tieren jeweils 1 ml des schädigenden Agens (100 %-iger Äthylalkohol, 0,6 n wäßrige Salzsaurelösung oder 0,2 n wäßrige Natriumhydroxid-Lösung) ebenfalls intragastral appliziert. Eine Stunde nach Applikation des schädigenden Agens werden die Tiere getötet. Bei der Autopsie werden das Ausmaß der in den Magen-/Darmwandungen auftretenden Läsionen beurteilt und deren Flächen durch computerisierte Planimetrie vermessen und als Prozent der glandulären Magenfläche berechnet.

In der nachfolgenden Tabelle II werden die mit Pinaveriumbromid erhaltenen Testergebnisse wiedergegeben.

**Tabelle II**

| Vorbehandlung mit Pinaveriumbromid | | Schädigendes Agens 1 ml | Schädigung: hämorrhagische Läsionen % der glandulären Magenfläche |
|---|---|---|---|
| Dosis mg/100g Ratte i.g. | Zeitspanne | | |
| 0 | 30 min | C₂H₅OH | 21,6±1,7 |
| 10 | 30 " | C₂H₅OH | 0 |
| 10 | 1 h | C₂H₅OH | 0 |
| 10 | 2 " | C₂H₅OH | 0,1±0,1 |
| 0 | 30 min | 0,6 n HCl | 17,6±2,7 |
| 10 | 30 min | 0,6 n HCl | 0,7±0,6 |
| 0 | 30 min | 0,2 n NaOH | 22,9±1,9 |
| 10 | 30 min | 0,2 n NaOH | 0,3±0,2 |

Die vorstehenden pharmakologischen Versuchsergebnisse zeigen, daß die Sübstanzen nach oraler Applikation eine starke, sowohl schnell einsetzende als auch langanhaltende Schutzwirkung gegenüber dem schädigenden Einfluß einer Vielzahl von chemischen Substanzen im gastrointestinalen Trakt entfalten und das Auftreten von hämorrhagischen Erosionen wirksam hemmen können.

Die Substanzen zeichnen sich durch eine Kombination von spasmolytischen Eigenschaften mit cytoprotektiven Wirkungen im Magen/Darmtrakt, insbesondere im mikrovaskulären Bereich der Magen-/Darmwände, aus und vermögen den Magen/Darmtrakt Darmtrakt vor durch gastrotoxische Dosen von Arzneimitteln, insbesondere NSAIDs, und anderen chemischen Substanzen induzierten hämorrhagischen Schäden zu schützen.

Die Substanzen können erfindungsgemäß als Wirkstoffe zur Herstellung von pharmakologischen Zubereitungen zur Prophylaxe und Behandlung von Blutzirkulationsstörungen im periphären, insbesondere mikrovaskulären Bereich der Magen-/Darmwände und von durch gastrotoxische Dosen von Arzneimitteln, insbesondere NSAIDs, oder Chemikalien induzierten Schädigungen der Magen-/Darmwände eingesetzt werden. Die zu verwendenden Dosen können individuell verschieden sein und variieren naturgemäß je nach Art des zu behandelnden Zustandes und der verwendeten Substanz. Im allgemeinen eignen sich zur oralen Applikation am Menschen und an größeren Säugetieren jedoch Arzneiformen mit einem Wirkstoffgehalt von 10 bis 100, insbesondere 20 bis 60 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als beispiele fester oral applizierbarer Präparate seien Tabletten, Kapseln, Granulate oder Dragees genannt. Diese Präparate können pharmazeutisch übliche anorganische und/oder organische Trägerstoffe wie z.B. Talkum, Milchzucker oder Stärke neben pharmazeutisch üblichen Hilfsmitteln, beispielsweise Gleitmitteln oder Tablettensprengmitteln, enthalten. Vorteilhafterweise werden die Präparate mit einem geschmackskaschierenden Überzug versehen. Flüssige Präparate wie Suspensionen oder Emulsionen der Wirkstoffe, vorzugsweise in mikroverkapselter Form, können die üblichen Verdünnungsmittel wie Wasser, Öle und/oder Suspensionsmittel wie Polyäthylenglycole u. dgl. enthalten. Es können zusätzlich weitere Hilfsstoffe zugegeben werden, wie z.B. Konservierungsmittel, Geschmackskorrigentien u. dgl.

Die Wirkstoffe können mit den pharmazeutischen Hilfs-und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden. Diese können gewünschtenfalls in an sich bekannter Weise dragiert werden.

Erfindungsgemäß werden die Substanzen insbesondere eingesetzt zur Prophylaxe und zur Behandlung von durch Einnahmen von NSAIDs hervorgerufenen gastrointestinalen Hämorrhagien, welche bis zu lebensbedrohlichen Magenblutungen insbesondere bei solchen Patienten führen können, welche über längere Zeit, beispielsweise im Rahmen einer Rheumatherapie, größere Mengen NSAIDs einnehmen. Erfindungsgemäß erweist es sich dementsprechend als zweckmäßig, bei länger anhaltenden Therapien mit NSAIDs eine Co-Medikation mit einer Verbindung der Formel I, insbesondere Pinaveriumbromid, durchzuführen. Durch eine kombinierte Anwendung eines NSAIDs mit einer Verbindung der Formel I, insbesondere Pinaveriumbromid, lassen sich die schädigenden Nebenwirkungen des NSAIDs auf den gastrointestinalen Trakt, insbesondere die Magenblutungen verursachenden Wirkungen, zurückdrängen und die Verträglichkeit einer NSAID-Therapie wesentlich verbessern. Das Mengenverhältnis zwischen dem Wirkstoff aus der Gruppe der NSAIDs und der Verbindung der Formel I kann je nach Art des eingesetzten NSAIDs variieren und kann beispielsweise zwischen 2 : 1 und 1 : 10, vorzugsweise 1 : 1 und 1 : 3 liegen. Die Wirkstoffe können getrennt in jeweils separaten pharmazeutischen Zubereitungen verabreicht werden, oder können gemeinsam in einer pharmazeutischen Zübereitung formuliert sein.

Die folgenden Beispiele sollen die Herstellung von Verbindungen der Formel I enthaltenden pharmazeutischen Zubereitungen näher erläutern, ohne jedoch den Umfang der Anmeldung zu begrenzen.

### Beispiel 1:

### Pinaverium enthaltende Tabletten

### Zusammensetzung:

### Pinaveriumbromid-Tabletten

### Herstellungsvorschrift:

50 Teile Pinaveriumbromid
80 Teile mikrokristalline Cellulose
17 Teile Maisstärke
18 Teile Lactose
1 Teil hydrophobes Siliciumdioxid
3 Teile Talkum
1,5 Teile Magnesiumstearat
Pinaveriumbromid wird mit Cellulose, Maisstärke, Lactose und Siliciumdioxid gemischt. Danach werden zu dieser Mischung Talkum und Magnesiumstearat zugemischt. Die so erhaltene Masse wird zu Tabletten von 170 mg verpreßt.

### Beispiel 2:

### Pinaveriumbromid-Filmtabletten

50 Teile Pinaveriumbromid
80 Teile mikrokristalline Cellulose
17 Teile Maisstärke
18 Teile Lactose
1 Teil hydrophobes Siliciumdioxid
8 Teile Talkum
1,5 Teile Magnesiumstearat
4,5 Teile Acrylharz
1 Teil Polyäthylenglykol
Pinaveriumbromid wird mit Cellulose, Maisstärke, Lactose und Siliciumdioxid gemischt. Danach werden zu dieser Mischung Talkum und Magnesiumstearat zugemischt. Die so erhaltene Masse wird zu 175 mg-Tabletten verpreßt. Die Tabletten werden mit einem magensaftlöslichen Filmüberzug bestehend aus Acrylharz, Polyäthylenglykol und Talkum versehen.

### Beispiel 3:

### Pinaveriumbromid-Tabletten

50 Teile Pinaveriumbromid
80 Teile mikrokristalline Cellulose
17 Teile Maisstärke
18 Teile Lactose
1 Teil hydrophobes Siliciumdioxid
3 Teile Talkum
1,5 Teile Magnesiumstearat
Pinaveriumbromid wird mit Cellulose, Lactose und 12 Teilen der Maisstärke gemischt. Die Mischung wird mit einer Paste aus 5 Teilen Maisstärke und 45 Teilen Wasser angeteigt und das so entstandene Granulat getrocknet. Nach Siebung des getrockneten Granulates werden Talkum, Siliciumdioxid und Magnesiumstearat zugemischt und diese Mischung zu Tabletten von 250 mg verpreßt.

### Beispiel 4:

### M Pinaveriumbromid-Filmtabletten

50 Teile Pinaveriumbromid
80 Teile mikrokristalline Cellulose
17 Teile Maisstärke
18 Teile Lactose
1 Teil hydrophobes Siliciumdioxid
3 Teile Talkum
1,5 Teile Magnesiumstearat
3 Teile Hydroxypropylmethylcellulose
1 Teil Polyäthylenglykol
Pinaveriumbromid wird mit Cellulose, Lactose und 12 Teilen der Maisstärke gemischt. Die Mischung wird mit einer Paste aus 5 Teilen Maisstärke und 45 Teilen Wasser angeteigt und das so entstandene Granulat getrocknet. Nach Siebung des getrockneten Granulates werden Talkum, Siliciumdioxid und Magnesiumstearat zugemischt und diese Mischung zu 170 mg-Tabletten verpreßt.

Die Tabletten werden mit einem magensaftlöslichen Filmüberzug bestehend aus Hydroxypropylmethylcellulose und Polyäthylenglykol versehen.

### Beispiel 5:

### Pinaveriumbromid-Kapseln

50 Teile Pinaveriumbromid
80 Teile mikrokristalline Cellulose
17 Teile Maisstärke
18 Teile Lactose
1 Teil hydrophobes Siliciumdioxid
3 Teile Talkum
1,5 Teile Magnesiumstearat
Pinaveriumbromid wird mit Cellulose, Lactose und 12 Teilen der Maisstärke gemischt. Die Mischung wird mit einer Paste aus 5 Teilen Maisstärke und 45 Teilen Wasser angeteigt und das so entstandene Granulat getrocknet. Nach Siebung des getrockneten Granulates werden Talkum, Siliciumdioxid und Magnesiumstearat zugemischt und diese Mischung in Hartgelatinekapseln einer gewünschten Größe abgefüllt.

Gewünschtenfalls kann das Granulat auch in der gewünschten Einzeldosis entsprechenden Portionen in Portionsbeutel (Sachets) abgefüllt werden.

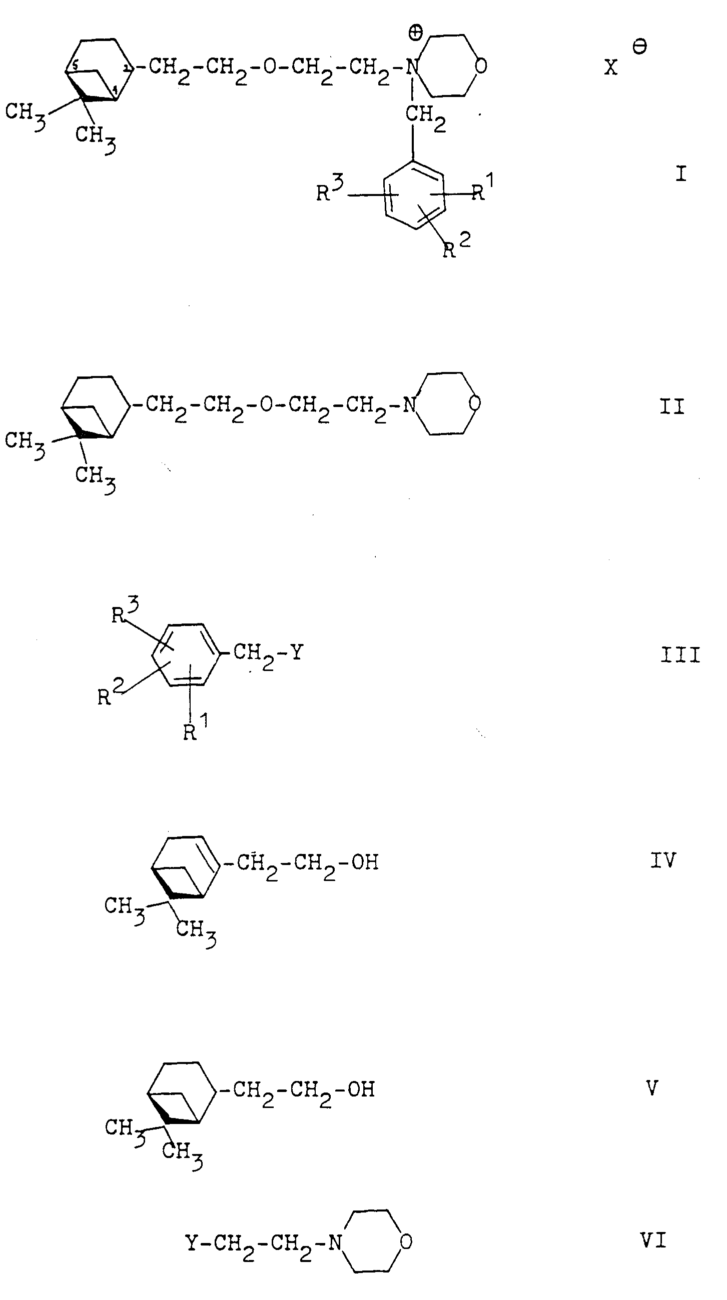

## Patentansprüche

1. Verwendung von quartären N-Benzyl-N-{2-[2-((1S,5S)-6,6-dimethylbicyclo[3.1.1]hept-2-yl)-äthoxy]-äthyl}-morpholinium-Salzen der allgemeinen Formel I 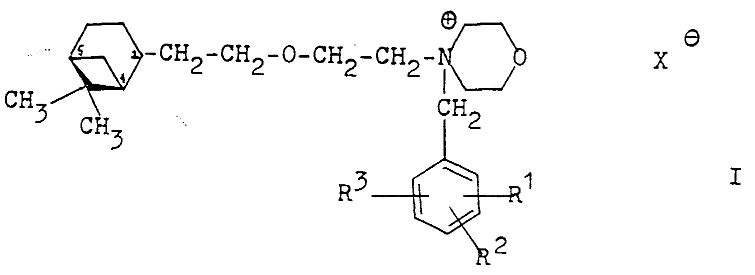 worin
R¹ Wasserstoff, Halogen, niederes Alkyl oder niederes Alkoxy bedeutet,
R² Wasserstoff, Halogen, niederes Alkyl oder niederes Alkoxy bedeutet,
R³ Wasserstoff, Halogen, niederes Alkyl oder niederes Alkoxy bedeutet und
X⁻ das Anion einer pharmakologisch akzeptablen Säure darstellt,
zur Herstellung von pharmazeutischen Zubereitungen zur Prophylaxe und Behandlung von Blutzirkulationsstörungen im peripheren, insbesondere mikrovaskulären Bereich der Magen-/Darmwände und von durch gastrotoxische Dosen von Arzneimitteln, insbesondere nichtsteroidalen entzündungshemmenden Arzneimitteln, oder Chemikalien induzierten Schädigungen der Magen-/Darmwände in größeren Säugetieren und Menschen.

2. Verwendung nach Anspruch 1 von solchen Salzen der Formel I, worin X⁻ das Anion einer Halogenwasserstoffsäure oder einer niederen aliphatischen oder aromatischen Sulfonsäure darstellt.

3. Verwendung nach Anspruch 1, dadurch gekennzeichnet, daß N-(2-Brom-4,5-dimethoxybenzyl)-N-{2-[2-((1S,5S)-6,6-dimethylbicyclo[3.1.1]hept-2-yl)-äthoxy]-äthyl}-morpholiniumsalze von pharmakologisch akzeptablen Säuren eingesetzt werden.

4. Verwendung von Salzen nach Anspruch 3, dadurch gekennzeichnet, daß Salze einer Halogenwasserstoffsäure oder niederen aliphatischen oder aromatischen Sulfonsäure eingesetzt werden.

5. Verwendung von Salzen nach Anspruch 4, dadurch gekennzeichnet, daß das Bromid eingesetzt wird.

## Claims

1. The use of quaternary N-benzyl-N-{2-[2-((1S,5S)-6,6-dimethylbicyclo-[3,1,1]hept-2-yl)-ethoxy]-ethyl}-morpholinium salts of the general Formula I, 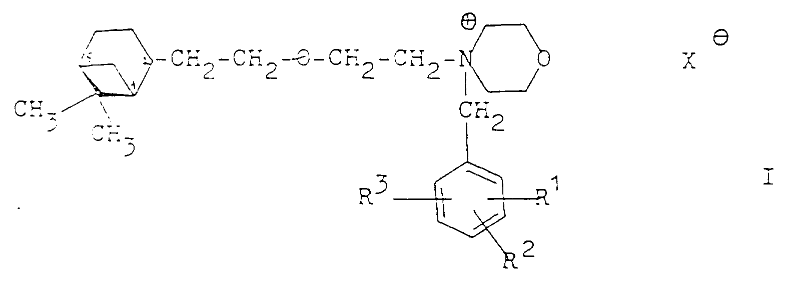 wherein
R¹ is hydrogen, halogen, lower alkyl or lower alkoxy,
R² is hydrogen, halogen, lower alkyl or lower alkoxy,
R³ is hydrogen, halogen, lower alkyl or lower alkoxy and
X⁻ represents the anion of a pharmacologically acceptable acid,
for the production of pharmaceutical preparations for prophylaxis and treatment of blood circulation disorders in the peripheral, in particular microvascular, region of the stomach/intestine walls and of damage to the stomach/intestine walls in larger mammals and humans, caused by gastrotoxic doses of medicaments, in particular non-steroid, anti-inflammatory medicaments, or chemicals.

2. The use according to Claim 1 of those salts of Formula I wherein X⁻ represents the anion of a hydrohalic acid or a lower aliphatic or aromatic sulphonic acid.

3. The use according to Claim 1, characterised in that N-(2-bromo-4,5-dimethoxybenzyl)-N-{2-[2-((1S,5S)-6,6-dimethylbicyclo[3,1,1]hept-2-yl)-ethoxy]-ethyl}-morpholinium salts of pharmacologically acceptable acids are used.

4. The use of salts according to Claim 3, characterised in that salts of a hydrohalic acid or lower aliphatic or aromatic sulphonic acid are used.

5. The use of salts according to Claim 4, characterised in that the bromide is used.

## Revendications

1. Utilisation de sels de N-benzyl-N-(2-[2-((1S,5S)-6,6-diméthylbicyclo[3.1.1]hept-2-yl)-éthoxy]-éthyl)-morpholinium quaternaires, de formule générale I : 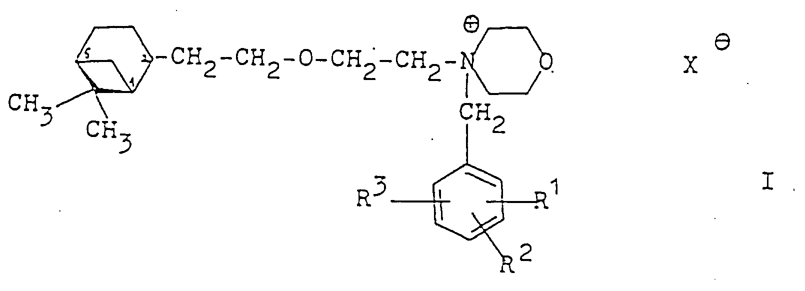 [dans laquelle
R¹ représente un atome d'hydrogène, un atome d'halogène, un groupe alkyle inférieur ou alcoxy inférieur;
R² représente un atome d'hydrogène, un atome d'halogène, un groupe alkyle inférieur ou alcoxy inférieur,
R³ représente un atome d'hydrogène, un atome d'halogène, un groupe alkyl inférieur ou alcoxy inférieur, et
X représente l'anion d'un acide pharmacologiquement acceptable],
pour la fabrication de préparations pharmaceutiques pour le traitement prophylactique et le traitement curatif de troubles de la circulation du sang dans les zones périphériques, notamment micro-vasculaires, de la paroi de l'estomac/intestin et pour traiter des affections de la paroi de l'estomac/intestin chez des mammifères supérieurs et chez des êtres humains, induites par des doses gastro toxiques de médicaments, en particulier des médicaments non stéroïdiens destinés à combattre une inflammation, ou induites par des produits chimiques.

2. Utilisation selon la revendication 1 des sels de formule I, dans laquelle X⁻ représente l'anion d'un hydracide halogéné ou d'un acide sulfonique aliphatique inférieur ou aromatique.

3. Utilisation selon la revendication 1, caractérisée en ce qu'on utilise des sels de N-(2-bromo-4,5-diméthoxybenzyl)-N-(2-[2-((1S,5S)-6,6-diméthylbicyclo [3.1.1] hept-2-yl)-éthoxy]-éthyl)-morpholinium d'acides pharmacologiquement acceptables.

4. Utilisation de sels selon la revendication 3, caractérisée en ce qu'on utilise des sels d'un hydracide halogéné ou d'un acide sulfonique aliphatique inférieur ou aromatique.

5. Utilisation de sels selon la revendication 4, caractérisée en ce qu'on utilise le bromure.
